# EUROPEAN PATENT SPECIFICATION

(11) **EP 3 485 531 B1**
(45) Date of publication and mention of the grant of the patent: **22.06.2022**
(21) Application number: 17737595.3
(22) Date of filing: 11.07.2017
(51) Int. Cl.: H01Q 1/22, H01Q 1/27, H04B 5/00, A61B 5/00, H01Q 5/328

(54) **MEDICAL IMPLANT WITH WIRELESS COMMUNICATION**
MEDIZINISCHES IMPLANTAT MIT DRAHTLOSER KOMMUNIKATION
DISPOSITIF D'IMPLANT MÉDICAL AVEC COMMUNICATION SANS FIL

(30) Priority: 13.07.2016 GB 201612175
(43) Date of publication of application: 22.05.2019
(73) Proprietor: Oslo Universitetssykehus HF, 0424 Oslo (NO)
(72) Inventor: KHALEGHI, Ali, 7053 Trondheim (NO); BALASINGHAM, Ilangko, 0489 Oslo (NO); BERGSLAND, Jacob, 1446 Drøbak (NO)
(74) Representative: Dehns
(86) International application number: PCT/EP2017/067461
(87) International publication number: WO 2018/011235

(56) References cited:
- WO-A1-2011/111008
- US-A1- 2004 257 220
- US-A1- 2006 097 918
- US-A1- 2009 005 656
- US-A1- 2009 043 183
- STEWART J THOMAS ET AL: "Modulated backscatter for ultra-low power uplinks from wearable and implantable devices", MEDICAL COMMUNICATION SYSTEMS, ACM, 2 PENN PLAZA, SUITE 701 NEW YORK NY 10121-0701 USA, 13 August 2012 (2012-08-13), pages 1-6, XP058008117, DOI: 10.1145/2342536.2342538 ISBN: 978-1-4503-1478-7
- KHALEGHI A ET AL: "Wireless communication link for capsule endoscope at 600 MHz", 2015 37TH ANNUAL INTERNATIONAL CONFERENCE OF THE IEEE ENGINEERING IN MEDICINE AND BIOLOGY SOCIETY (EMBC), IEEE, 25 August 2015 (2015-08-25), pages 4081-4084, XP032811068, DOI: 10.1109/EMBC.2015.7319291

## Description

The present invention relates to a medical implant arranged for wireless communication from within the body, and to a system for communication with the implant, as well as to related methods.

Medical implants are used to gather information about the body and to interact with the body in various contexts. For example, capsule endoscopes are used to gather images within the digestive systems as well as to obtain samples or deliver drugs, neural prosthetic systems link the brain with external devices and exchange electrical signals with the brain, pacemakers can be implanted, and various other devices have been proposed that rely on being held within the body or passed through the body. For all of these medical implant devices it provides advantages if there can be wireless communication with other devices outside of the body. This may be for wireless control or programming of the implant, for transmission of data from sensors such as cameras, temperature sensors, blood monitoring sensors and the like, and other things.

Typical data transfer rates and the depth of the implant beneath the body surface are set out in the table below. Wireless data transmission within these ranges is highly desirable in order to allow for fully wireless operation of the implant. In fact for some applications it is impossible to have a wired connection from out of the body to the implant, and difficult as well as inconvenient to have a wired link to a separate implanted transmitter device closer to the body surface (as is used in some cases for neural prosthetics).

| Application | Required data rate | Range |
|---|---|---|
| **Capsule endoscope (video streaming)** | 1-100 Mbps | 30-150 mm |
| **Heart monitoring** | 40-200 Kbps | 100 mm |
| **Neural monitoring** | 512 sensors, 430 Mbps | 20-30 mm |

For a fully wireless connection from outside the body to the location of the implant device challenges can be generated both by the need for a minimum data transfer rate and by the depth of body tissue.

Implant devices with wireless communication capabilities and also wireless power transfer have been proposed in the prior art. For example, US 2013/0215979 describes a method for simultaneous power and data transfer from the exterior of the body to an implant device. A circuit at the implant device is used for power harvesting from the exterior RF signal and there may be backscatter communication from the implant to the exterior device. Load modulation is proposed for transmitting a data signal back to the exterior device. A part of the charging RF signal is used for backscatter communication and the remainder is used for the wireless power transfer.

US 2014/0055088 describes a method for wireless charging of an implant device. The backscatter from the communication coil is used to indicate the best frequency for efficient charging. Thus the external transmitter can adjust its frequency for wireless power transfer based on a rudimentary feedback mechanism using backscattered information.

US 2014/0084855 discusses wireless power transfer and data transmission to an implant (from exterior to implant). A backscatter signal is received in the exterior system and is processed in order to either control the implant impedance matching unit or to alter the frequency of the exterior device. In both cases this is done in order to obtain maximum wireless power transfer.

US 2009/043183 A1 relates to an implant device for use within the body and US 2009/005656 A1 relates to a modulated backscatter circuit for a medical implant.

None of these existing devices can provide the required data transfer rates and also penetrate sufficiently deep into the body. There is a need for a more effective way to communicate with medical implants, and in particular to increase the possible depth of body tissue for which communications can be effective, whilst at the same time avoiding undue use of the implant device's internal power supply. The ability to communicate reliably with implant devices at locations deeper within the body would greatly enhance the utility of wireless data transmission for medical implants.

Viewed from a first,exemplary aspect, which includes features not required by claim 1, the present disclosure provides an implant device for use within the body, the implant device comprising: a data source; and an antenna for backscatter communications with an external communication system; wherein the implant device is arranged to control the backscattering properties of the antenna in order to thereby transmit data from the data source to the external communications system; and wherein the antenna has a configuration for backscatter communications that comprises a meandering path extending from a start of the meander at a capacitive coupling fed by a coupling patch to an end of the meander connected to a ground plane.

With this shape and structure of antenna the inventors have found that the power of the backscattered data signal for a given incoming transmission signal can be maximised. This feature, in combination with the use of an external transmitter in the backscattered communication, means that the range of the antenna can be made a maximum without any impact on the power taken from an internal power source of the implant device. The external communication system is outside of the body and there are fewer constraints on the power available at the external communications system than at the implant device within the body. The strength of the power transmission can hence be increased compared to non-backscatter wireless transmissions using a transmitter at the implant within the body. By using an antenna with the proposed form then the strength of the 'reflected' signal used for the backscatter communication can be enhanced compared to other antenna shapes, such as coil antennas. Notably, the antenna optimised in this way differs from antennas optimised for wireless power transfer as used in the prior art that focusses on wireless power transmission. The proposed antenna can also be simply re-designed to operate in any intended frequency and the size of the implant device can be easily managed for any specific application since the antenna has a compact form relative to its effective cross-section for backscatter communications.

The ground plane that is connected to the end of the meander is a ground plane for the antenna, and typically is provided by a ground plane of the implant device.

The antenna may be capable of assuming multiple different configurations with changes in geometry as described below, or in a simpler implementation it may have just one main configuration optimised for backscatter communications. The length of the meandering path for the backscattering configuration of the antenna is set based on the desired resonant frequency of the antenna, which itself may be set based on the frequency used for transmissions by the external communication system. The meander length may extend from a start point adjacent the capacitive coupling patch to an end point at the connecting point to the ground. A meander length of about 0.4 A (A is the wavelength) is required to provide the resonant frequency at f=c/A in free space. However, the exact resonance length of the meander depends on the meander strip width, the size of a coupling gap to the coupling patch and the distance between the meander lines. The exact resonance length for a given antenna design may be determined by simulating the geometry using full wave electromagnetic techniques.

The meandering path preferably comprises multiple turns of a serpentine nature, for example there may be ten or more turns joined by a corresponding number of meander lines, optionally fifteen or more turns. The meandering path may take the shape of a square wave, optionally with the amplitude (the 'height' of meander lines) of the square wave being greater than the wave length (the spacing between sets of meander lines). The amplitude of the square wave may be three or more times the wavelength of the square wave, for example about four times the wavelength, or more. Other waveforms with a similar ratio of amplitude to wavelength could be used.

The antenna could be implemented with the meander formed on a flat planar surface. An antenna of this type would have low thickness but relatively large length and width, which may be suitable for some implant geometries. Alternatively, where a more compact antenna will be beneficial then the antenna may be implemented with the meander formed on a three dimensional shape, for example about the walls of a prism, including on curved walls of a prism. One example is implemented with the meander formed on the wall of a cylindrical prism. Such an arrangement has a greater minimum dimension than the planar arrangement, but a smaller maximum dimension giving a more compact arrangement, which can be advantageous for medical implants, especially implants that are mobile within the body such as capsule endoscopes.

The implant device is a medical implant device and it includes sensors for gathering data for medical use, such as use in medical diagnostic methods or for medical research. The sensors are preferably connected to the data source in order to provide information used to generate the data that is transmitted from the data source to the external communication system. The implant device may additionally includes a memory for data storage, and may also include additional data processing devices such as a computer processor. The implant device is a capsule device for use in the human digestive system, such as a capsule endoscope. It is particularly advantageous to increase the possible maximum depth of tissue for wireless data transmission in the case of implants travelling through the digestive system, since the digestive system can be deep beneath lossy tissues.

As noted above it has been proposed in the prior art to combine backscatter communications with wireless power transfer. Using wireless power transfer reduces the range for backscatter within the body since the antenna must necessarily absorb some of and often a major part of the transmitted energy. With the present antenna the design of the antenna in the backscatter communication configuration is for maximum reflection and hence minimal absorption of energy, and the implant device is preferably arranged to use as much as possible of the energy incident on the antenna for backscatter communications. The implant device may be arranged such that there is no wireless power transfer during communication of data via the antenna. Optionally there is never any wireless power transfer, although as noted below in some instances there may be a separate use of wireless power transfer when backscatter communication is not being carried out.

The data source or sensors of the implant may have their own battery or a separate wireless power transfer system. The data source may be arranged to provide a data signal for controlling the backscattering properties of the antenna to thereby transmit data from the data source to the external communication system. Preferably this is a low power data signal thereby ensuring maximum lifetime for the implant device. By minimising the power requirement at the implant device the lifespan of the device is maximised whilst also allowing for a high data rate of the backscatter communications.

The implant device may be arranged to use any suitable technique for controlling the backscattering properties of the antenna. Advantages arise from the particular geometry of the antenna for the implant device with any type of backscattering control. The implant device may use load modulation, for example. In some examples, which provide additional advantages, the implant device uses one or more switching devices for controlling the backscattering properties, preferably low power electrical switching devices such as transistors. For example, Field-effect transistors (FET)s such as CMOS FETs may be used. In one example the transistor is a CMOS MMIC device that advantageously can operate with ultra low power. The use of a transistor provides a low power way to alter the backscatter properties of the antenna. The implant device may include one or more switches to change the load on the antenna and/or change the antenna structure. The use of switches in this way in order to enable low power control of backscatter communications at an implant device is considered novel and inventive in its own right.

Hence, the present invention provides an implant device for use within the body, the implant device comprising: a data source; and an antenna for backscatter communications with an external communication system; wherein the implant device is arranged to control the backscattering properties of the antenna in order to thereby transmit data from the data source to the external communications system; wherein the backscattering properties of the antenna are controlled by one or more electrical switch(es) that change the load and/or geometry of the antenna; wherein the implant device is a medical implant device and includes one or more sensor(s) for gathering data for medical use and a memory for data storage; and wherein the implant device is a capsule device for use within the human digestive system.

With this combination of features the antenna can be controlled with very low power required from the implant device and with the possibility of a high change in antenna cross-section, which allows for effective backscattering communication. Preferably this feature is combined with an antenna geometry that has a high maximum antenna cross-section, in particular the antenna with a meandering path as set out above in connection with the first exemplary aspect of the disclosure. The implant device of the second aspect may also or alternatively be combined with any of the other features described above, either with or without including the meandering path geometry for the antenna.

An internal power source of the implant device may be used to control the electrical switch(es) as well as also powering the data source. This may be a battery or optionally it may be a wireless power transfer system that is separate to the antenna used for backscatter communications. The implant device preferably does not take power from the antenna during the backscattered communication, which ensures that the antenna can be optimised for maximum reflection and hence maximum range for the backscatter communications with the external communication systems.

There may be multiple electrical switches having differing effects on the antenna's backscattering properties and hence allowing for more than two different states for the backscattered signal. This can allow for more complicated data transfer and higher data rates.

At least one switch of the one or more switches may have the effect of changing the load on the antenna. For example, the switch may alternate between a high impedance connection such as an open circuit and a low impedance connection such as a short circuit, and this switch may be located at a feed point of the antenna. In one example the antenna uses a capacitive coupling to a coupling patch and the switch alternates between a high impedance connection and a low impedance connection between the coupling patch and a ground plane, which may be the ground plane mentioned above in the first aspect.

At least one switch of the one or more switches may have the effect of changing the geometry of the antenna. For example, the switch may alternate between a high impedance connection such as an open circuit and a low impedance connection such as a short circuit, and this switch may be located midway along a length of the antenna. In one example the at least one switch alternates between a high impedance connection and a low impedance connection between a ground plane, which may be the ground plane mentioned above in the first aspect, and a point midway along the length of the antenna, optionally there may be more than one switch with these switches spaced apart along the length of the antenna. As set out above the length of the antenna affects its resonant frequency and since the switch effectively changes the length of the antenna by changing the point at which the antenna is coupled to the ground plane then this has a significant effect on the backscatter properties of the antenna.

An example antenna has a configuration for backscatter communications as in the first aspect and thus comprises a meandering path extending from a start of the meander at a capacitive coupling fed by a coupling patch to an end of the meander connected to a ground plane of the device. With this arrangement there may be a switch at a feed point to the coupling patch and/or there may be a switch, optionally multiple switches, at points along the length of the antenna.

Changes to the antenna geometry change the resonant frequency and can optimise the antenna performance for differing purposes. Advantageously, the use of switches to adjust the length of the antenna means that the antenna can be used with multiple resonant frequencies. One use for this is to allow the antenna to have one or more configuration(s) for backscatter communications, which may involve transmitted radio waves at different frequencies. Another use for this is to allow for a wireless power transfer configuration of the antenna, where the antenna geometry and/or the antenna resonant frequency is more suited to wireless power transfer than the backscattering configuration(s) of the antenna. This means that the antenna can be used at separate times, and in differing configurations, either for backscattering communication or for wireless power transfer. The implant device may be arranged to use the wireless power transfer configuration as an internal power source for the implant device and/or to charge an internal battery that provides an internal power source for the implant device.

The features set out below may be used in relation to either of the first and second aspect, or indeed to an implant device combining features of both the first and second aspect.

The implant device may be provided in combination with an external communication system that interacts with the implant device to receive data from the implant device. For example, this may be in the context of a system for gathering medical data about a patient. The external communication system is arranged to transmit an electromagnetic wave toward the implant device, and to receive a backscattered signal from the implant device. This may be done using a single antenna acting as both transmitter and receiver, but preferably the external communication system includes separate transmitting and receiving antenna. One reason for using multiple antennas is to reduce the coupling between the transmission antennas and receiving antennas in order to prevent the receiver saturation. This allows the receiver to operate in its full dynamic range for receiving the relatively weak backscatter signals. In addition, in case of multipath propagation, using bi-static (dual antennas) or multi-static (multiple antennas) performs better than mono-static (single antenna) configurations as this removes the probability of deep signal fading.

The electromagnetic wave may be a radio wave and in particular may be at a frequency designated for use with medical implants. Such frequencies are selected based on their availability and loss characteristics in living tissues. The Industrial, Scientific and Medical (ISM) frequency band, and Medical Implant Communication Service (MICS) at 400, 600, 800 and 1400 MHz are used for wireless communication with medical implants and the external communication system may hence use one of these frequencies.

Due to the material properties of body tissues the resonant frequency of the antenna is shifted downwards compared to the resonant frequency in free space. This resonance shift depends on the material properties of the implant location. For example, fat tissues cause a smaller shift than tissues with greater water content such as blood or muscle. As the exact resonant frequency is not known and can vary then the transmitter and receiver of the external communication system advantageously may be arranged to seek the relevant frequency for best communication and to lock on to that frequency. The resonant frequency of the antenna may also change as it passes through the body tissue. The transmitter searches for the best frequency with maximum backscatter performance. The search algorithm is based on a feedback from the receiver. The reason for the frequency search is that the resonance of the antenna that is altered by the tissues' loading.

In a typical situation the antenna resonant frequency when in body may be about 35% below the resonant frequency for the same antenna in free space. In addition to this shift in resonance, the antenna resonance inside body varies by about +/-5% to +/- 10% depending on the implant's surrounded material (fat, muscle, and other liquids). The frequency search tries to maximize the backscatter signal by tuning to the antenna resonance.

The transmitter may be arranged to adjust its transmission frequency whilst the external communications system monitors the reception of the backscattered signal at the receiver, with the intention of setting a frequency which provides the best reception of the backscatter signal from the implant device antenna at the receiver. This may be done by transmitting a signal at varying frequency and identifying the frequency with the best reception at the receiver. The frequency may, for example, be swept through a band of ±5% or ±10% around the nominal transmission frequency, which may be one of the frequencies mentioned above.

The receiver is arranged to adjust its resonant frequency to match the frequency of the backscattered signal, i.e. to match its frequency to the transmission frequency, which has been optimised for maximum backscattering.

The transmitter may be arranged to emit multiple electromagnetic waves having multiple different frequencies, and the receiver may be arranged to receive at the same multiple different frequencies. Thus, there may be multiple transmitting antennas and multiple receiving antennas with these antennas configured to operate at differing frequencies, or alternatively the transmitter and/or the receiver antenna may be an antenna capable of operating effectively with varying frequencies. The use of multiple frequencies can allow for increased rates of data transfer during the backscatter communication, and advantageously may be combined with an implant device having an antenna that can be configured with multiple resonant frequencies, for example by the use of switches that change the length of the antenna as discussed above. Any suitable circuit may be used for processing of the received signal. With the use of multiple frequencies the data transmission can be similar to binary phase shift keying (BPSK) modulation, with a correspondingly reduced error rate.

The transmitter may comprise an on body antenna as the transmitter antenna. This may be a loop antenna such as a circular or square loop. There may be multiple transmitting antennas at multiple locations acting together in order to transmit the electromagnetic wave toward the implant device. The use of multiple antennas at different locations can allow the signal strength at the implant device to be maximised whilst avoiding excessive levels of electromagnetic radiation at the body surface, thus increasing the potential range for the backscatter communications without exceeding limits on the exposure of the patient to radiation.

The transmitting antenna(s) may be placed directly on the body, preferably with a gap of less than 1 mm between the electrical parts of the antenna and the skin. Alternatively, or additionally, a matching layer may be placed between the antenna(s) and the skin. The matching layer may comprise a low loss dielectric material, for example water or an aqua based dielectric material. With the use of a matching layer the body Specific Absorption Rate (SAR) is reduced and heat generated due to the radiation can be dissipated. Other loop antenna configurations may also be implemented. With the use of a loop antenna the SAR due to the electric field is reduced.

In some implementations, the external communications system may be arranged to reduce the amount of the coupled power at the receiver antenna due to the transmitter antenna by using multiple transmitter antennas to make a null coupled signal at the receiver. This means that even a very weak backscatter signal can be recovered at receiver. This further increases the communication range of the setup by involving the receiver in its full dynamic range

The implant device as in the first aspect may be used in order to gather medical information about a patient or to treat the patient. The device may include any of the other features discussed above.

The use of the device may comprise: implanting or inserting the implant device into the patient; transmitting an electromagnetic wave toward the device from an external communication system that is outside of the body; controlling the backscattering properties of the antenna in order to thereby transmit data from the data source using the backscattered signal from the antenna; and receiving the backscattered signal at the external communications system.

Optionally, the use of the device may include using the implant device to gather medical information at one or more locations within the patient's body, and then transmitting this information out of the body via the backscatter communications with the external communication system. The use of the device may further include controlling the backscatter properties of the antenna to transmit data via the backscatter signal by means of switches as described above.

Certain preferred embodiments of the present invention will now be described by way of example only and with reference to the accompanying drawings, in which:
Figure 1 is a schematic diagram showing communications between an implant device and a communications system outside of the body;
Figures 2a to 2c show an antenna for the implant device as a schematic in a) flat and b) tubular form, and c) in actual size;
Figures 3a and 3b show the antenna of Figures 2a and 2b when the antenna structure is reconfigured to change the backscattered signal;
Figures 4a and 4b show an antenna with multiple switch locations allowing for multiple configurations of the antenna structure in a) schematic and b) actual size;
Figure 5 is a diagram of a scheme for using two different carrier frequencies for communications with the implant device;
Figure 6a and 6b show scatter plots for a) single frequency communication and b) dual frequency communication;
Figure 7 is a plot of bit error rate (BER) for single and dual frequency communication; and
Figure 8 shows possible geometries for an on-body antenna used to transmit a signal to the implant device.

Wireless communications with medical implant devices can be conducted by using electromagnetic waves, but there are a number of challenges with this. Using an implant device as the transmitter of information consumes a significant amount of power at the implant since there is a large path loss associated with wave propagation inside body tissues. The implant should typically be small and power resources at the implant are therefore very restricted. It is not desirable to consume a large part of the energy from the implant for data transmission and the need to do so can reduce the implant lifetime. Some applications such as neuronal implants, capsule endoscopes, and heart implants require continuous data transfer from the implant device. Therefore, the implant lifetime is important. The data rate can be limited in some kilobits per second to provide longer operation period. However, the quality of the service is lessened and the operation time is limited for several hours. Using backscatter communications minimises the power required from the implant device for data transmissions since the transmitter is moved outside of the body.

Different frequency bands are allocated for implant application. These frequencies are selected based on their availability and loss characteristics in living tissues. The Industrial, Scientific and Medical (ISM) frequency band, and Medical Implant Communication Service (MICS) at 400, 600, 800 and 1400 MHz are used for wireless communication with medical implants. The material properties of human tissues are frequency dependent. The conductivity of human tissues increases with frequency increment that hampers electromagnetic wave propagation for higher frequencies. If the frequency increases above 1.5 GHz, the path loss increases exponentially. The frequency band at 400 and 600 MHz allows the minimum path loss through human tissues that can have potential application for deep implants.

Due to the inherently lossy characteristics of living tissues and the limited power resources of an implant device, continuous data transmitting with high-speed characteristics becomes difficult. The transmitted power to and from the implant is also limited by EM exposure standards where the internal organ Specific Absorption Rate (SAR) limits the transmitted power.

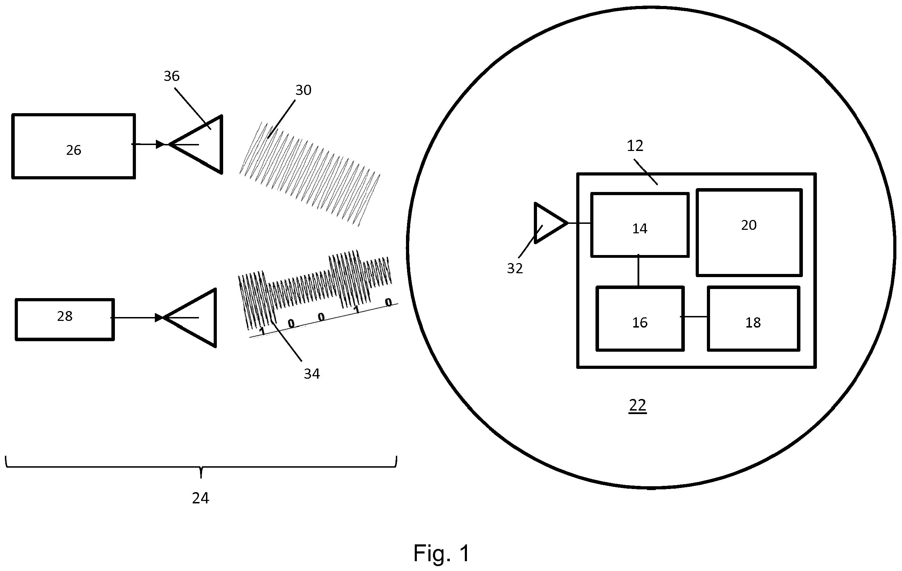

As shown in Figure 1, it is proposed to use a backscatter technique for reading the information from an implant device 12 within the body 22. The implant device 12 contains an antenna 14 and the data source 16, which is attached to implant systems 18 such as sensors. The implant device 12 can also include a battery 20 for providing power to the sensors 18 in order that the sensors 18 can be used at all times, i.e. without the need for wireless transmission of power. If wireless transmission of power is used then this is done at a different time to the use of backscatter communications so as to ensure the maximum strength of backscatter signals. The lifetime of the implant device 12 is extended compared to conventional devices since the battery 20 is not used to power data transmission out of the body. The technique used for data transmission is a radar approach in which the external communications system 24 includes both the transmitter 26 and receiver 28. Thus, the power resources at the implant 12 are not constrained.

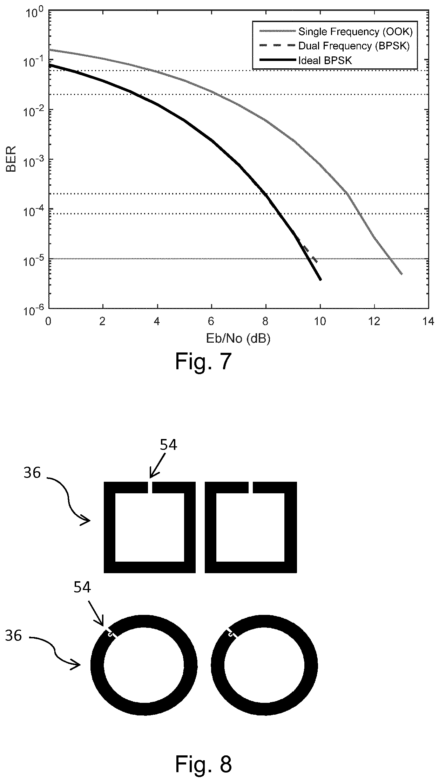

The transmitter 26 sends a signal 30 at a suitable frequency for transmission through body tissue, for example 800 MHz. The frequency of the transmitted signal 30 is set based on the geometry of the antenna 32 of the implant device 12, with an allowance for frequency shift due to the lossy nature of the body tissue. It will be appreciated that the antenna 32 could easily be adapted for use with different frequencies. The reflected signal 34 from the implant device 12 contains information from the data source 16 transmitted by reconfiguration of the implant antenna 32 as explained in more detail below. The information in the reflected signal 34 is extracted at the receiver 28 outside of the body 22. In this technique the internal organ SAR is almost eliminated and the SAR issue is shifted to the body surface on which the transmitter antenna 36 is located. On the body surface, due to the available space, it is more convenient to control the SAR by using an appropriate design for the transmitter antenna 36, as explained below with reference to Figure 8.

In the system of Figure 1 a continuous radio wave signal 30 is transmitted from the outside body toward the implant device 12 via the on-body antenna 36. Body tissues absorb the wave 30, and the signal strength is reduced.. The implanted antenna 32, depending on its size and geometry, reflects a part of the incident wave. The wave is reflected back and the reflected wave 34 is transmitted through the body organs and is received by the transmitting antenna 36 or more preferably by a second on-body antenna connected to the receiver 28. By providing variations in the implant antenna 32 reflection characteristics of the reflected signal 34 is modulated in order to transmit information from the data source 16 to the receiver 28.

A difficulty with using the backscatter technique for a medical implant arises due to the path loss, which is not a one-way propagation. The radio wave 30 must penetrate into depth to the implant device 12 through high loss medium of body tissues 22 and should be reflected back 34 and then received by the on-body receiver 28. Therefore, the path loss contains two ways of wave propagation. Thus, larger transmitting power than the one-way propagation is required to compensate the extra path loss. However, the energy source for the off-body device 24 is not limited and therefore it is easy to transmit the original radio wave 30 with significant power in order to compensate for the path loss. On the body surface, the power limitation is governed with the SAR term which is maximum at the body surface in the proximity of the transmitter antenna 36. The other main limitation is the implant backscatter antenna 32. The implanted antenna 32 must provide high reflection to the incident field despite its small size. It must be conformal and consistent with the size of the implant device 12 and any other restrictions on the shape of the implant device 12.

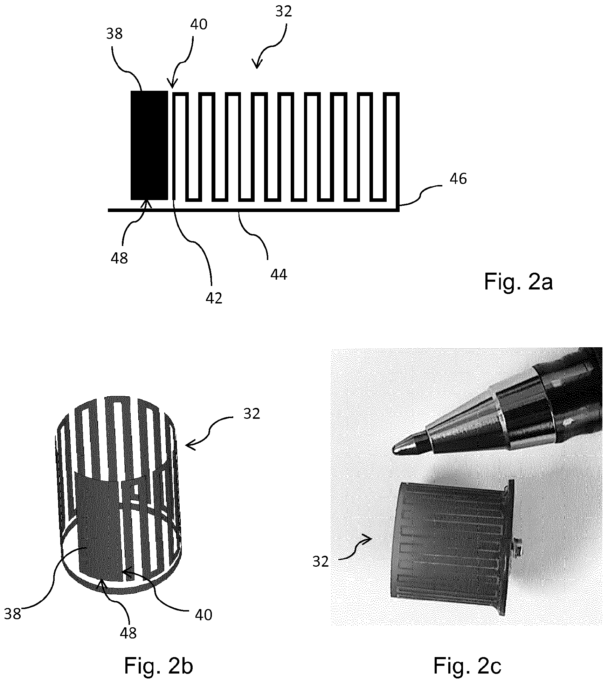

The example implantable antenna 32, as shown in Figure 2a, is a meander-shaped structure that is fed using a coupling patch 38 via a coupling gap 40 at the start 42 of the meander. The meander antenna 32 is connected to the ground plane 44 of the implant device 12 at the end 46 of the meander and it is powered using the capacitive coupling mechanism with the patch metal strip 38. This antenna 32 can provide an impedance of 50 Ohms at its resonant frequency if it is powered from the patch centre point 48 as indicated. This impedance matches the requirement for connecting any transmitter/receiver system to the antenna 32.

The resonant frequency of the antenna 32 feeding on the feed point 48 is determined by the meander length, which is counted from the start point 42 beside the rectangular patch 38 to the end point 46 at the connecting point to the ground 44. A meander length of about 0.4 A (A is the wavelength) is required to provide the resonant frequency at f=c/A in free space. As explained above, the resonant frequency differs with the presence of body tissue compared to when the antenna is in free space. In addition, the exact resonance length of the meander depends on the meander strip width, the size of the coupling gap 40 to the rectangular patch 38 and the distance between the meander lines. These parameters can be used to adjust the feed impedance to other source impedances rather than the 50 Ohms of this example. The exact resonance length for a given antenna design can be determined by simulating the geometry using full wave electromagnetic techniques.

The antenna 32 can be manufactured by PCB printing technology on top of dielectric materials. Using flexible PCB material for manufacturing of the antenna or 3D printing of the antenna on a conformal geometry is also feasible. For instance, the antenna can be formed in a cylinder shape as shown in Figures 2b and 2c. Thus, the antenna 32 can be made more compact with a cylindrical geometry that with the equivalent flat geometry. The void within the centre of the cylindrical antenna 32 could be used for other components of the implant device 12. The antenna resonant frequency remains almost unchanged despite the change in the shape of the antenna 32 since the resonance is determined by the meander length.

For conducting backscatter based communication, two main objectives should be fulfilled. The first one is to generate significant Antenna Cross Section (ACS) which can guarantee larger power reflections from the antenna 32. This requirement is necessary to enhance the communication link in the backward channel. The second requirement is to generate significant differential ACS (ΔACS) so that the receiver 28 can easily separate out different states for the backscattered signal. To produce a large ΔACS the antenna impedance for the frequency in which the communication is conducted must be altered in a way that the difference between ACSs for two states of the antenna impedances becomes significant. This is what enables the receiver 28 to distinguish the ACS change when the structure of the antenna 32 is manipulated by the data source 16.

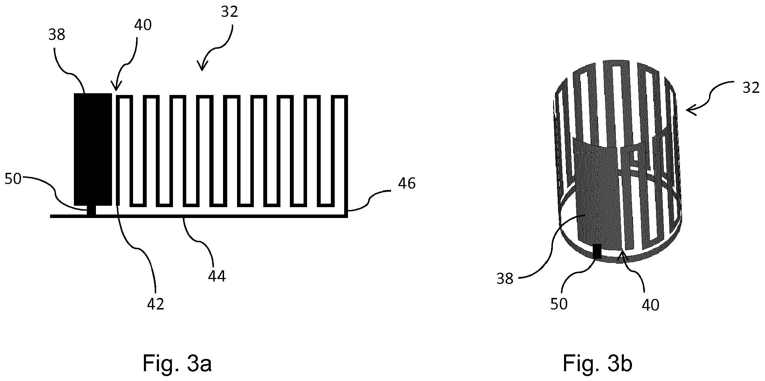

To provide large ACS for backscatter communications the feed point 48 of the designed antenna 32 is connected to the system ground plane 44 via a short circuit 50 as shown in Figures 3a and 3b. Using this technique, the ACS is at a maximum compared to loading the feed point 48 with other impedances. Thus, with the feed point impedance at z=0 Ohms then maximum ACS is achieved at the resonant frequency, which is defined by the meander antenna length. The value of ACS is maximum at the antenna resonant frequency, and its amount is related to the normalized dimension of the antenna to the wavelength in the operating environment. Therefore, for a given frequency large antenna size can generate larger ACS compared to small size antennas.

To accomplish the second task with providing large ΔACS we have several alternatives. One approach is to make a switching between two states of loads at the feed point 48. One state should be a short circuit 50 or very low impedance to generate significant ACS. The other load can be simply an open circuit state in which the feed point 48 is left in the air with no connection of the patch 38 to the ground 44. Using an open circuit the antenna resonance is moved significantly to higher frequencies. Thus, the antenna becomes non-resonating at the first frequency and the ACS becomes very low at the first resonant frequency. Therefore, large differences of ACS are achieved for the two given load states at the first resonant frequency. To accomplish the data communication operation using the backscatter approach a switching circuit is used in which the switching between the two states of open circuit and short circuit 50 at the feed point 48 are controlled by the data source 16 at the implant 12. The transmitter 26 at the antenna resonant frequency radiates an electromagnet wave toward the backscatter antenna 32, and the switch state defines the level of reflection. The receiver 28 receives the reflected signal and the data can be extracted using an Amplitude Shift Keying (ASK) demodulator scheme.

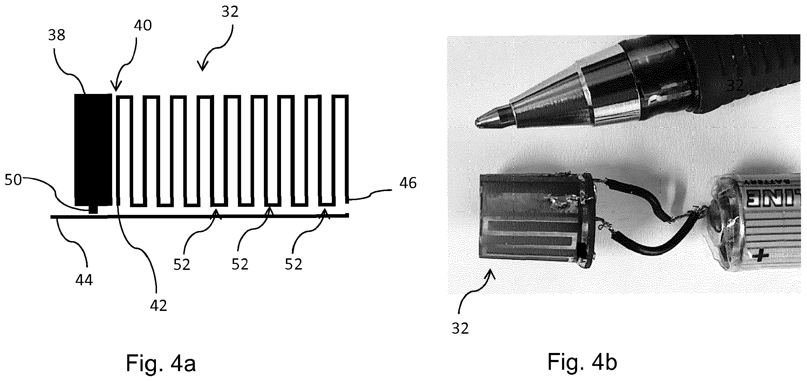

Using the above switching method of short circuit 50 and open circuit at the feed point 48, the location of the second resonant frequency is not easy to control. To be able to control the second resonance of the antenna 32 we can apply the switching operation along the meander geometry to the ground plane 44 or other load impedances in multiple specified locations 52 in the meander structure, as shown in Figures 4a and 4b. The antenna geometry provides resonance related to the length of the meander shape. If the meander is connected to the ground plane in locations 52 other than the meander end 46, the antenna resonant frequency is shifted upward as the electrical length of the meander shortens. Therefore, by modifying the geometry the antenna 32 can have a reconfigurable and adjustable resonant frequency. Advantageously, the adjustment in geometry and in antenna length can also allow for one configuration of the antenna 32 to be optimised for receiving wireless power transmissions, for example to recharge the internal battery or to boost the power available for gathering information via the sensors 18. The same antenna 32 structure can be used to provide for both a maximum strength of backscattered signal in one configuration, and effective harvesting of wireless power transmissions in another configuration. Different frequencies of radio wave can be used for the wireless power transmission compared to for the backscatter communication since the resonant frequency of the antenna 32 can be adjusted by changing the length of the meander.

Having a significant minimum shift among the resonances is necessary to provide larger ΔACS. The frequency shift should be greater than the bandwidth of the reflected data signals which is defined by the data rate and the bit shaping scheme in the switching circuit. A switching bank (not shown) at the transmitter 26 can be used to generate multiple pre-designed resonant frequencies.

For best performance as an implanted antenna 32 in biological living tissues the antenna should have a container to protect it from the environment. A gap of 0.2 mm around the antenna is required to reduce the environment effects on the antenna performance.

Due to the material properties of the tissues surrounding the antenna 32 and the implant device 12, the resonant frequency is shifted downwards. This resonance shift depends on the material properties of the implant location. For example, fat tissues cause a smaller shift than tissues with greater water content such as blood or muscle. As the exact frequency shift is not known, the transmitter and receiver system of the backscatter communication scheme has the option to seek the resonant frequency and lock on to that frequency. Experimental results have indicated a downward frequency shift of about 30% for the implanted antenna compared to the same antenna in the air.

In the example of Figure 1 a single carrier frequency is used to illuminate the antenna 32. By using single carrier transmission, it is possible to have an on-off keying (OOK) modulation of the data in which the presence of the reflections shows one state of data and the other state is the non-reflecting condition.

As noted above, with the addition of further switches at locations 52 along the length of the meander structure the antenna 32 can switch between multiple resonances with the data stream. In this approach, multiple carrier frequencies are used to illuminate the antenna 32 in place of the single carrier frequency from the single transmitter 26 of Figure 1. For instance, consider two transmitted frequencies at the two resonances of the geometry of the antenna 32 and two receivers 28, which are adjusted to decode the data in the transmitted carrier frequencies. The data reading in the backscatter channel can be conducted in two independent channels, separately. The data in two channels are opposite of each other because only one of the carrier frequencies is reflected back in the in relation to the switch state. By using this approach, the OOK backscatter modulation is realized, and we can provide a redundant link for the backscatter communication.

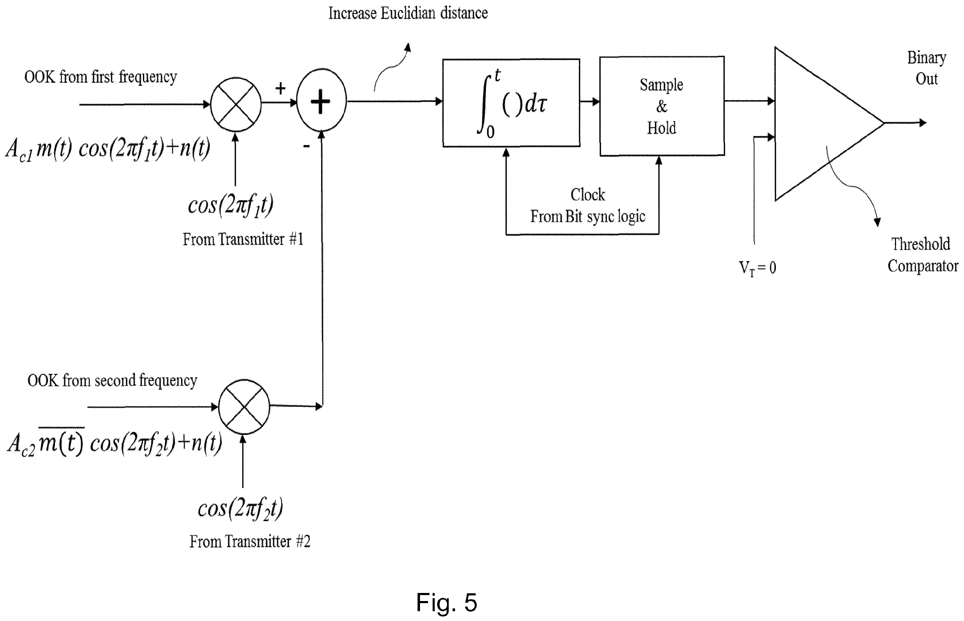

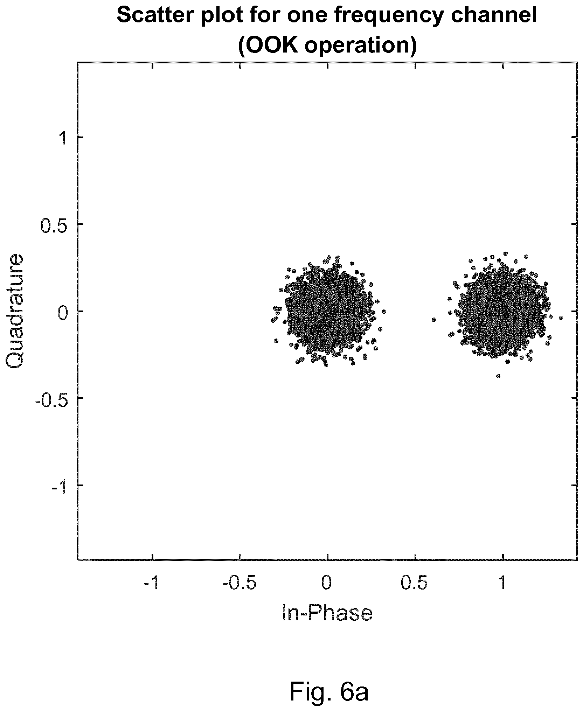

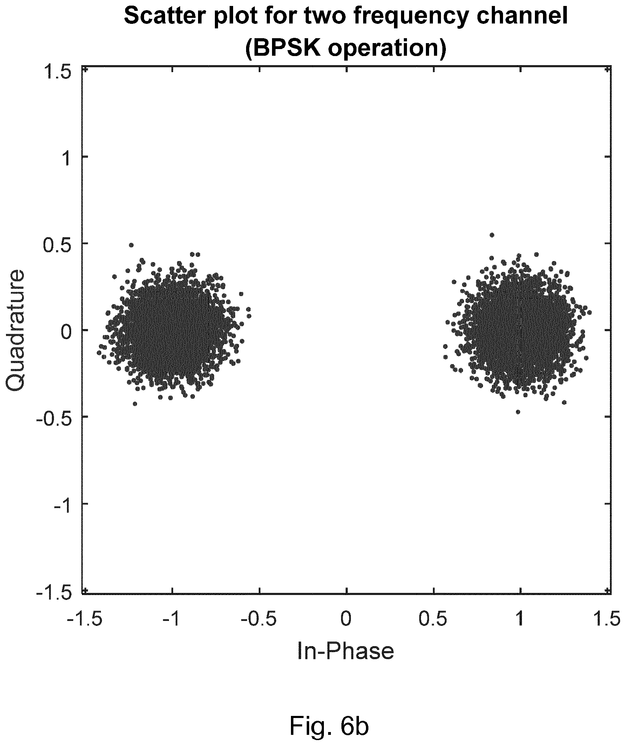

We propose a scheme in which we can improve the bit-error-rate (BER) performance using two carrier's data reading as shown in Figure 5. As we know, the reflections in two resonant frequencies are opposite of each other. In fact, for a given instant we have the reflection of "0" state in one frequency and the reflection of "1" state in the other frequency. Therefore using the difference between the two received signals in the two frequency channels increase the Euclidian distance of data and thus, the system can perform similarly to binary phase shift keying (BPSK) modulation instead of the OOK in the single carrier usage. Figure 6 shows the scatter plot of data for each channel observation and the combined channel using the scheme in Figure 5 and Figure 7 shows the BER performance. The BER performance for an ideal BPSK demodulation is shown for comparison.

The on-body antenna 36 is an important part of the communication link for backscatter application. The antenna 36 of the transmitter 26 must provide a significant amount of energy to the implant device 12 and the same antenna or another antenna should be able to receive the reflected signal. The transmitter antenna 26 can be a printed strip loop antenna with a circular or square form as shown in Figure 8. A loop antenna is a form of a radiation element with a closed conductor in the current path. The loop antenna is used as the source for generating the magnetic fields, which have a lesser loss than the electric field in body tissues. This antenna technique can deliver excellent signal quality for implant in the backscatter link.

The loop antenna 36 can be in different forms, for example circular or rectangular curls as shown in Figure 8. Each loop antenna 36 has a feed point 54, as shown. The loop impedance depends on the antenna diameter and the strip width. For an on-body application, we need small antenna configuration. To provide small and high efficient antenna 36, the loop is printed on a flexible substrate material. The impedance of the antenna 36 is increased and matched to the source impedance of 50 ohms by attaching the substrate layer of the antenna 36 to the body surface. Thus, the on-body antenna 36 is touched to the body surface. As there would be variable fat and skin layers for different patients, a matching layer is used below the antenna. This can be a low loss dielectric layer between the antenna 36 and the body surface, for example a container holding a low loss dieletric material such as water or an aqua based material. With this technique, the antenna matching to source 50 Ohms impedance is provided despite the small diameter of the loop. For instance, the loop diameter of about λ/15 is used instead of λ/2. By using water or other high permittivity liquids, the heat which is generated by the loop antenna is delivered to the dielectric instead of the body tissues. The SAR value is reduced relatively. Thus, more power can be used for the backscatter operation

The compact size of the proposed transmitter antenna 36 provides simple attachment to the body surface. By using this antenna 36, the path-loss for the designed implant antenna 32 in a depth of 70 mm inside the body is about 40 dB.

The on body antenna 36 for backscatter applications can be in the form of mono-static, bi-static or multi-static. The mono- static configuration has a single antenna 36 for the transmitter and the receiver in which a circulator separates the transmit and receive paths. The bi-static or multi-static configuration has, at least, two or more antennas in which one antenna 36 is used for transmitting, and the other antennas for receiving. An array of the loop antennas can be used on the body surface in the form of a belt, and the communication is performed by transmitting with one of the antennas 36 and receiving the reflected signal via one of the next antennas on the body surface. Switching among the transmit and receive antennas is conducted to find the best backscatter signal from the implant device 12. It is notable that the coupling between two side by side antennas is significantly high compared to the antennas apart from each other. The strong coupling limits the receiver sensitivity as the receiver would saturate. Thus, the backscatter signals which are weak become difficult for detection. There are several known techniques to reduce the coupling among antennas. One simple way is to use antennas at a distance of about 8 cm on the body surface.

The implanted antenna 32 is a resonator inside the human body tissues 22. The resonant frequency of the antenna 32 alters during the antenna movement inside different body tissues. A frequency shift of about 10% in various tissues inside the body is expected. The on-body transmitter system 26 should adjust its transmission frequency to receive the maximum backscatter signal from the implanted antenna 32. Thus, the on-body system 24 has the feature of tracking for the best frequency and looking for the appropriate frequency for the optimized backscatter link. An example encapsulated antenna 32 operates at 800 MHz in the air and its frequency shifts to around 555 MHz with 10% variation in different internal organs. This operating frequency is pre-defined, and the out of body system 24 can apply frequency tuning of 10% to improve the link by about 4 dB.

In certain scenarios, the antenna orientation or the antenna surrounding materials would cause strong signal attenuation in the backscatter link. In this situation, the second alternative frequency (that is pre-defined with the antenna design) can be used for the communication purpose. Thus, the system should switch to the other frequency band to establish the communications if it is found that there are problems with the first resonant frequency.

Considering a scenario with multiple transmitter antennas 36, the communication link can be established by switching among the transmitter and the receiver antennas to establish the backscatter communications. With N number of transmitter antennas that could establish communication with the backscatter device, then multi-antenna focusing is possible. In this approach an improved link quality can be achieved. In one possible scheme, a set of phase shifters is assigned to each transmitter chain with its antenna, which can be a loop antenna 36 as discussed above. The antenna(s) that provided the backscatter in the selected receiver 28 are used. The following algorithm is applied to focus transmitted carriers into the implant antenna and thus, improve the backscatter link.
1- The first transmitter 26 is applied and the backscatter data signal is detected.
2- The second transmitter 26 comes into operation, and the phase of the carrier is swept in the range of 0 to 180 degrees until the data signal quality maximizes.
3- The next transmitters 26 are sequentially comes to the operation, and the step 2 is repeated.

Using the above procedure N number of transmitter signals are focussed spatially at the implant antenna 32, and the link quality improves by several dBs (maximum 6 dB for using two transmitters, 3 dB because of the power and 3 dB because of the array processing gain). In this case, it is possible to reduce the radiated power from the on body transmitter antennas 36 and this results in reduced exposure (SAR) for the patient.

By adding the multiple transmitters 26 into the circuit and following the algorithm above, it is also feasible to lessen the direct coupling of the intense carrier signal (which has no information inside) in the receiver chain. In this situation, the receiver electronics operates in full sensitivity range, and the BER performance is ameliorated because of the reduced interference signal i.e. increased signal to noise interference ratio.

## Claims

1. An implant device (12) for use within the body (22), the implant device comprising:
a data source (16); and
an antenna (32) for backscatter communications with an external communication system;
wherein the implant device (12) is arranged to control the backscattering properties of the antenna (32) in order to thereby transmit data from the data source (16) to the external communications system;
wherein the backscattering properties of the antenna (32) are controlled by one or more electrical switch(es) configured to
change the load and/or geometry of the antenna (32)
wherein the implant device (12) is a medical implant device and includes one or more sensor(s) (18) for gathering data for medical use and a memory for data storage; and
wherein the implant device (12) is a capsule device for use within the human digestive system.

2. The implant device (12) as claimed in claim 1, wherein the antenna (32) has a configuration for backscatter communications that comprises a meandering path extending from a start of the meander (42) at a capacitive coupling fed by a coupling patch (38) to an end of the meander (46) connected to a ground plane and preferably wherein in the backscattering configuration of the antenna (32) the length of the meandering path from a start point adjacent the capacitive coupling patch to an end point at the connecting point to the ground plane is set based on the desired resonant frequency of the antenna (32).

3. The implant device (12) as claimed in any preceding claim, wherein the switch(es) are low power electrical switching devices such as transistors.

4. The implant device (12) as claimed in any preceding claim, wherein the device comprises multiple electrical switches configured to have differing effects on the antenna's backscattering properties and hence allowing for more than two different states for the backscattered signal.

5. The implant device (12) as claimed in any preceding claim, wherein at least one switch of the one or more switches is configured to have the effect of changing the load on the antenna by alternating between a high impedance connection such as an open circuit and a low impedance connection such as a short circuit, and this switch is located at a feed point of the antenna.

6. The implant device (12) as claimed in any preceding claim, wherein at least one switch of the one or more switches is configured to have the effect of changing the geometry of the antenna by alternating between a high impedance connection such as an open circuit and a low impedance connection such as a short circuit, and this switch is located midway along a length of the antenna between a ground plane and the point midway along the length of the antenna.

7. The implant device (12) as claimed in any preceding claim, wherein at least two switches of the one or more switches are configured to have the effect of changing the geometry of the antenna by alternating between a high impedance connection such as an open circuit and a low impedance connection such as a short circuit, and these switches are spaced apart along the length of the antenna and connect between the antenna and a ground plane.

8. The implant device (12) as claimed in any preceding claim, wherein the one or more sensor(s) are connected to the data source in order to provide information used to generate the data that is transmitted from the data source to the external communication system.

9. The implant device (12) as claimed in any preceding claim, wherein the data source and/or the one or more sensor(s) of the implant device are powered by a battery or a separate wireless power transfer system.

10. The implant device (12) as claimed in any preceding claim, wherein the implant device is arranged such that there is no wireless power transfer to the implant antenna during backscatter communication of data using the implant antenna.

11. The implant device (12) as claimed in any preceding claim, wherein the data source is arranged to provide a data signal for controlling the backscattering properties of the antenna to thereby transmit data from the data source to the external communication system.

12. A combination of the implant device (12) as claimed in any preceding claim and an external communication system that is arranged to transmit an electromagnetic wave from a transmitter (26) toward the implant device and to receive a backscattered signal from the implant device at a receiver (28) and preferably wherein the electromagnetic wave is a radio wave at a frequency approved for use with medical implants.

13. The combination as claimed in claim 12, wherein the transmitter (26) of the external communication system is arranged to adjust its transmission frequency whilst the external communications system monitors the reception of the backscattered signal at the receiver (28), with the intention of setting a frequency which provides the best reception of the backscatter signal from the implant device antenna at the receiver, and/or, wherein the receiver (28) is arranged to adjust its operating frequency to match the frequency of the backscattered signal.

14. The combination as claimed in claim 12 or 13, wherein the external communication system is arranged to emit multiple electromagnetic waves having multiple different frequencies and to receive backscatter communications from the implant device (12) at multiple different frequencies.

15. The combination as claimed in any of claims 12 to 14, wherein the transmitter (26) comprises an on body loop antenna (36), and preferably comprising multiple transmitting antennas (36) at multiple locations on the body configured to act together in order to transmit the electromagnetic wave toward the implant device, and more preferably wherein the external communications system is arranged to reduce the amount of the coupled power at a receiver (28) antenna due to the transmitter antenna by using the multiple transmitter antennas (36) to make a null coupled signal at the receiver.

## Patentansprüche

1. Implantatvorrichtung (12) zur Verwendung innerhalb des Körpers (22), wobei die Implantatvorrichtung eine Datenquelle (16); und
eine Antenne (32) für Rückstreuungskommunikation mit einem externen Kommunikationssystem umfasst;
wobei die Implantatvorrichtung (12) eingerichtet ist, um die Rückstreuungseigenschaften der Antenne (32) zu steuern, um dadurch Daten von der Datenquelle (16) an das externe Kommunikationssystem zu senden;
wobei die Rückstreuungseigenschaften der Antenne (32) durch einen oder mehrere elektrische Schalter gesteuert werden, welche konfiguriert sind, um die Last und/oder Geometrie der Antenne (32) zu ändern,
wobei die Implantatvorrichtung (12) eine medizinische Implantatvorrichtung ist und einen oder mehrere Sensor(en) (18) zum Erfassen von Daten zur medizinischen Verwendung und einen Speicher zur Datenspeicherung beinhaltet; und
wobei die Implantatvorrichtung (12) eine Kapselvorrichtung zur Verwendung innerhalb des menschlichen Verdauungssystems ist.

2. Implantatvorrichtung (12) nach Anspruch 1, wobei die Antenne (32) eine Konfiguration für Rückstreuungskommunikation aufweist, welche einen mäandernden Pfad umfasst, welcher sich von einem Startpunkt des Mäanders (42) an einer kapazitiven Kupplung, welche durch eine Kupplungsverbindung (38) gespeist wird, zu einem Endpunkt des Mäanders (46) erstreckt, welcher mit einer Massefläche verbunden ist, und wobei bevorzugt in der Rückstreuungskonfiguration der Antenne (32) die Länge des mäandernden Pfades von einem Startpunkt neben der kapazitiven Kupplungsverbindung zu einem Endpunkt an dem Verbindungspunkt mit der Massefläche auf Basis der gewünschten Resonanzfrequenz der Antenne (32) festgelegt wird.

3. Implantatvorrichtung (12) nach einem der vorstehenden Ansprüche, wobei der/die Schalter elektrische Niederleistungsschaltvorrichtungen wie beispielsweise Transistoren sind.

4. Implantatvorrichtung (12) nach einem der vorstehenden Ansprüche, wobei die Vorrichtung mehrere elektrische Schalter umfasst, welche konfiguriert sind, um unterschiedliche Effekte auf die Rückstreuungseigenschaften der Antenne aufzuweisen und somit mehr als zwei verschiedene Status für das rückgestreute Signal zu erlauben.

5. Implantatvorrichtung (12) nach einem der vorstehenden Ansprüche, wobei zumindest ein Schalter des einen oder der mehreren Schalter konfiguriert ist, um den Effekt des Änderns der Last für die Antenne durch Wechseln zwischen einer Hochimpedanzverbindung wie beispielsweise einem offenen Stromkreis und einer Niederimpedanzverbindung wie beispielsweise einem Kurzschluss aufzuweisen, und dieser Schalter sich an einem Speisepunkt der Antenne befindet.

6. Implantatvorrichtung (12) nach einem der vorstehenden Ansprüche, wobei zumindest ein Schalter des einen oder der mehreren Schalter konfiguriert ist, um den Effekt des Änderns der Geometrie der Antenne durch Wechseln zwischen einer Hochimpedanzverbindung wie beispielsweise einem offenen Stromkreis und einer Niederimpedanzverbindung wie beispielsweise einem Kurzschluss aufzuweisen, und dieser Schalter sich in der Mitte entlang einer Länge der Antenne zwischen einer Massefläche und dem Punkt in der Mitte entlang der Länge der Antenne befindet.

7. Implantatvorrichtung (12) nach einem der vorstehenden Ansprüche, wobei zumindest zwei Schalter des einen oder der mehreren Schalter konfiguriert sind, um den Effekt des Änderns der Geometrie der Antenne durch Wechseln zwischen einer Hochimpedanzverbindung wie beispielsweise einem offenen Stromkreis und einer Niederimpedanzverbindung wie beispielsweise einem Kurzschluss aufzuweisen, und diese Schalter entlang der Länge der Antenne beabstandet sind und zwischen der Antenne und einer Massefläche verbinden.

8. Implantatvorrichtung (12) nach einem der vorstehenden Ansprüche, wobei der eine oder die mehreren Sensor(en) mit der Datenquelle verbunden sind, um Informationen bereitzustellen, welche verwendet werden, um die Daten zu erzeugen, welche von Datenquelle an das externe Kommunikationssystem gesendet werden.

9. Implantatvorrichtung (12) nach einem der vorstehenden Ansprüche, wobei die Datenquelle und/oder der eine oder die mehreren Sensor(en) der Implantatvorrichtung durch eine Batterie oder einen separaten drahtlosen Leistungsübertragungssystem gespeist werden.

10. Implantatvorrichtung (12) nach einem der vorstehenden Ansprüche, wobei die Implantatvorrichtung so eingerichtet ist, dass es keine drahtlose Leistungsübertragung zu der Implantatantenne während Rückstreuungskommunikation von Daten unter Verwendung der Implantatantenne gibt.

11. Implantatvorrichtung (12) nach einem der vorstehenden Ansprüche, wobei die Datenquelle eingerichtet ist, um ein Datensignal zum Steuern der Rückstreuungseigenschaften der Antenne bereitzustellen, um dadurch Daten von der Datenquelle an das externe Kommunikationssystem zu senden.

12. Kombination der Implantatvorrichtung (12) nach einem der vorstehenden Ansprüche und eines externen Kommunikationssystems, welches eingerichtet ist, um eine elektromagnetische Welle von einem Sender (26) hin zu der Implantatvorrichtung zu senden, und um ein rückgestreutes Signal von der Implantatvorrichtung in einem Empfänger (28) zu empfangen, und wobei bevorzugt die elektromagnetische Welle eine Funkwelle mit einer zur Verwendung in medizinischen Implantaten zugelassenen Frequenz ist.

13. Kombination nach Anspruch 12, wobei der Sender (26) des externen Kommunikationssystems eingerichtet ist, um seine Sendefrequenz anzupassen, während das externe Kommunikationssystem den Empfang der rückgestreuten Signals in dem Empfänger (28) überwacht, mit der Absicht, eine Frequenz festzulegen, welche den besten Empfang des Rücksstreuungssignals von der Implantatvorrichtungsantenne in dem Empfänger bereitstellt, und/oder wobei der Empfänger (28) eingerichtet ist, um seine Betriebsfrequenz anzupassen, damit sie mit der Frequenz des rückgestreuten Signals übereinstimmt.

14. Kombination nach Anspruch 12 oder 13, wobei das externe Kommunikationssystem eingerichtet ist, um mehrere elektromagnetische Wellen mit mehreren verschiedenen Frequenzen abzustrahlen, und um Rückstreuungskommunikation von der Implantatvorrichtung (12) auf mehreren verschiedenen Frequenzen zu empfangen.

15. Kombination nach einem der Ansprüche 12 bis 14, wobei der Sender (26) eine Schleifenantenne am Körper (36) umfasst, und bevorzugt mehrere Senderantennen (36) an mehreren Orten auf dem Körper umfasst, welche konfiguriert sind, um gemeinsam zu agieren, um die elektromagnetische Welle hin zu der Implantatvorrichtung zu senden, und wobei bevorzugter das externe Kommunikationssystem eingerichtet ist, um die Menge der gekoppelten Leistung in einer Antenne des Empfängers (28) aufgrund der Antenne des Sender durch Verwendung der mehreren Senderantennen (36) zu reduzieren, um im Empfänger ein gekoppeltes Nullsignal zu erzeugen.

## Revendications

1. Dispositif d'implant (12) pour utilisation dans le corps (22), le dispositif d'implant comprenant une source de données (16) ; et
une antenne (32) pour communications par rétrodiffusion avec un système de communication externe ;
dans lequel le dispositif d'implant (12) est conçu pour commander les propriétés de rétrodiffusion de l'antenne (32) afin de transmettre ainsi des données de la source de données (16) au système de communication externe ;
dans lequel les propriétés de rétrodiffusion de l'antenne (32) sont commandées par un ou plusieurs commutateurs électriques configurés pour changer la charge et/ou la géométrie de l'antenne (32)
dans lequel le dispositif d'implant (12) est un dispositif d'implant médical et inclut un ou plusieurs capteurs (18) pour regrouper des données en vue d'une utilisation médicale et une mémoire pour stockage de données ; et
dans lequel le dispositif d'implant (12) est un dispositif à capsule pour utilisation dans le système digestif humain.

2. Dispositif d'implant (12) selon la revendication 1, dans lequel l'antenne (32) présente une configuration pour communications par rétrodiffusion qui comprend un chemin sinueux s'étendant depuis un début du méandre (42) au niveau d'un couplage capacitif alimenté par une pièce de couplage (38) jusqu'à une fin du méandre (46) connectée à un plan de masse et de préférence dans lequel, dans la configuration de rétrodiffusion de l'antenne (32), la longueur du chemin sinueux d'un point de départ adjacent à la pièce de couplage capacitif à un point de fin au niveau du point de connexion au plan de masse est définie sur la base de la fréquence de résonance souhaitée de l'antenne (32).

3. Dispositif d'implant (12) selon une quelconque revendication précédente, dans lequel le(s) commutateur(s) sont des dispositifs de commutation électrique à faible énergie tels que des transistors.

4. Dispositif d'implant (12) selon une quelconque revendication précédente, dans lequel le dispositif comprend de multiples commutateurs électriques configurés pour présenter des effets différents sur les propriétés de rétrodiffusion de l'antenne et permettant donc plus de deux états différents pour le signal rétrodiffusé.

5. Dispositif d'implant (12) selon une quelconque revendication précédente, dans lequel au moins un commutateur des un ou plusieurs commutateurs est configuré pour présenter l'effet de changer la charge sur l'antenne en alternant entre une connexion à impédance élevée telle qu'un circuit ouvert et une connexion à faible impédance telle qu'un court-circuit, et ce commutateur est situé au niveau d'un point d'alimentation de l'antenne.

6. Dispositif d'implant (12) selon une quelconque revendication précédente, dans lequel au moins un commutateur des un ou plusieurs commutateurs est configuré pour présenter l'effet de changer la géométrie de l'antenne en alternant entre une connexion à impédance élevée telle qu'un circuit ouvert et une connexion à faible impédance telle qu'un court-circuit, et ce commutateur est situé à mi-chemin sur une longueur de l'antenne entre un plan de masse et le point à mi-chemin sur la longueur de l'antenne.

7. Dispositif d'implant (12) selon une quelconque revendication précédente, dans lequel au moins deux commutateurs des un ou plusieurs commutateurs sont configurés pour présenter l'effet de changer la géométrie de l'antenne en alternant entre une connexion à impédance élevée telle qu'un circuit ouvert et une connexion à faible impédance telle qu'un court-circuit, et ces commutateurs sont espacés sur la longueur de l'antenne et établissent une connexion entre l'antenne et un plan de masse.

8. Dispositif d'implant (12) selon une quelconque revendication précédente, dans lequel les un ou plusieurs capteurs sont connectés à la source de données afin de fournir des informations utilisées pour générer les données qui sont transmises de la source de données au système de communication externe.

9. Dispositif d'implant (12) selon une quelconque revendication précédente, dans lequel la source de données et/ou les un ou plusieurs capteurs du dispositif d'implant sont alimentés par une batterie ou un système de transfert d'énergie sans fil séparé.

10. Dispositif d'implant (12) selon une quelconque revendication précédente, dans lequel le dispositif d'implant est agencé de telle sorte qu'il n'y a pas de transfert d'énergie sans fil à l'antenne d'implant durant la communication par rétrodiffusion de données à l'aide de l'antenne d'implant.

11. Dispositif d'implant (12) selon une quelconque revendication précédente, dans lequel la source de données est conçue pour fournir un signal de données pour commander les propriétés de rétrodiffusion de l'antenne afin de transmettre ainsi des données de la source de données au système de communication externe.

12. Combinaison du dispositif d'implant (12) selon une quelconque revendication précédente et d'un système de communication externe qui est conçu pour transmettre une onde électromagnétique depuis un émetteur (26) vers le dispositif d'implant et pour recevoir un signal rétrodiffusé provenant du dispositif d'implant au niveau d'un récepteur (28) et de préférence dans lequel l'onde électromagnétique est une onde radio à une fréquence approuvée pour utilisation avec des implants médicaux.

13. Combinaison selon la revendication 12, dans laquelle l'émetteur (26) du système de communication externe est conçu pour ajuster sa fréquence de transmission tandis que le système de communication externe surveille la réception du signal rétrodiffusé au niveau du récepteur (28), avec l'intention de régler une fréquence qui fournit la meilleure réception du signal rétrodiffusé depuis l'antenne de dispositif d'implant au niveau du récepteur, et/ou, dans lequel le récepteur (28) est conçu pour ajuster sa fréquence de fonctionnement pour qu'elle corresponde à la fréquence du signal rétrodiffusé.

14. Combinaison selon la revendication 12 ou 13, dans laquelle le système de communication externe est conçu pour émettre de multiples ondes électromagnétiques présentant différentes fréquences et pour recevoir des communications par rétrodiffusion depuis le dispositif d'implant (12) à de multiples fréquences différentes.

15. Combinaison selon l'une quelconque des revendications 12 à 14, dans laquelle l'émetteur (26) comprend une antenne cadre sur corps (36), et comprenant de préférence de multiples antennes de transmission (36) à de multiples emplacements sur le corps configurées pour agir ensemble afin de transmettre l'onde électromagnétique vers le dispositif d'implant, et plus préférentiellement dans lequel le système de communication externe est conçu pour réduire la quantité de l'énergie couplée au niveau d'une antenne de récepteur (28) en raison de l'antenne d'émetteur en utilisant les multiples antennes d'émetteur (36) pour créer un signal couplé nul au niveau du récepteur.
